# EUROPEAN PATENT SPECIFICATION

(11) **EP 1 338 281 B1**
(45) Date of publication and mention of the grant of the patent: **22.03.2006**
(21) Application number: 01930355.1
(22) Date of filing: 24.04.2001
(51) Int. Cl.: C12N 15/10, A61K 31/70, A61K 31/711, A61K 33/24, A61K 33/26, A61K 35/60, A61K 35/14, A61K 35/28, A61K 35/55, C07H 21/04, C07H 21/00, A61P 31/14

(54) **METHOD FOR PRODUCING AN IMMUNOTROPIC ANTIVIRAL PREPARATION**
METHODE ZUR HERSTELLUNG EINER IMMUNOTROPISCHEN ANTIVIRALEN ZUSAMMENSETZUNG
PROCEDE DE FABRICATION D'UN PRODUIT ANTIVIRAL IMMUNOTROPIQUE

(30) Priority: 01.12.2000 RU 2000130036
(43) Date of publication of application: 27.08.2003
(73) Proprietor: Kaplina, Elli Nikolaevna, Moscow, 101000 (RU); Ladygina, Alexandra Yurievna, Moscow, 101000 (RU); Kaplin, Valery Yurievich, Moscow, 119136 (RU); Vainberg, Mihail Yurievich, Moscow, 125591 (RU)
(72) Inventor: Kaplina, Elli Nikolaevna, Moscow, 101000 (RU); Ladygina, Alexandra Yurievna, Moscow, 101000 (RU); Kaplin, Valery Yurievich, Moscow, 119136 (RU); Vainberg, Mihail Yurievich, Moscow, 125591 (RU)
(74) Representative: Sternagel, Fleischer, Godemeyer & Partner
(86) International application number: PCT/RU2001/000171
(87) International publication number: WO 2002/043740

(56) References cited:
- EP-A- 0 712 936
- EP-A1- 0 818 202
- WO-A-94/25473
- WO-A1-93/10793
- RU-C1- 2 043 767
- RU-C1- 2 108 797
- RU-C1- 2 136 286
- RU-C1- 2 158 592
- SU-A3- 1 833 547
- US-A- 4 861 759
- US-A- 5 547 684
- US-A- 5 662 889
- DATABASE WPI Section Ch, Week 200114 Derwent Publications Ltd., London, GB; Class B04, AN 2001-135552 XP002267635 & RU 2 158 592 C (KAPLINA E N), 10 November 2000 (2000-11-10)
- DATABASE WPI Section Ch, Week 199324 Derwent Publications Ltd., London, GB; Class B04, AN 1993-196720 XP002267636 & WO 93 10793 A (KAPLINA E N), 10 June 1993 (1993-06-10)
- DATABASE WPI Section Ch, Week 199618 Derwent Publications Ltd., London, GB; Class B04, AN 1996-178395 XP002267637 & RU 2 041 885 C (FARMZASHCHITA RES PRODN CENTRE), 20 August 1995 (1995-08-20)
- DATABASE WPI Section Ch, Week 199649 Derwent Publications Ltd., London, GB; Class B04, AN 1996-496098 XP002267638 & RU 2 055 837 C (FARMZASHCHITA RES PRODN CENTRE), 10 March 1996 (1996-03-10)
- DATABASE WPI Section Ch, Week 200035 Derwent Publications Ltd., London, GB; Class B04, AN 2000-409355 XP002267639 & RU 2 136 286 C (KAPLINA E N), 10 September 1999 (1999-09-10)

## Description

### Field of the invention

The invention relates to chemical and pharmaceutical industry and medicine, namely, to the method of obtaining immunotropic preparation possessing high antiviral activity towards RNA- and DNA-viruses, this preparation being obtained from animal material (soft roe of sturgeon fish or salmon fish, calf thymus, chicken blood, pig spleen).

### Background

The method of obtaining antiviral preparations from Na-DNA by thermolysis in acid medium, or by alkaline hydrolysis with 0.3N KOH is known. DNA destruction and denaturation occurs in both cases that not only leads to decrease in pharmacological activity, but provides no stability and reproducibility of the effect (DE 3724951, 1989).

There is known the method of obtaining DNA-based antiviral preparation according to which peroxide, hydroperoxide, chlorates, permanganates, diazoamino-compounds are added to the reaction at the stage of sonicating when high-polymer DNA degrades (RU 2108797, 1998). Disadvantage of this method consists in DNA degradation and denaturation that leads to decrease in biological activity and pharmacological unstability of the target product.

The method of obtaining RNA-DNA hybrid possessing immunotropic antiviral activity (RU 2108796, 1998) is known. However the said property of this hybrid is weak because of related RNA-DNA activity.

The method of obtaining the agent possessing antiviral activity on the basis of DNA sodium salt complex with metals (SU 1833547, 1991) is the most similar to the suggested invention. However the description of the method of obtaining antiviral complex does not disclose the essence of the process.

### Disclosure of the invention

The technical result of this invention consists in obtaining immunotropic antiviral pharmaceutical preparation possessing high therapeutic effect towards both RNA- and DNA-viruses.

The essence of the invention consists in achievement of the named technical result and in the method of obtaining immunotropic antiviral preparation based on native desoxyribonucleic acid sodium salt (DNA-Na). According to this method defatted soft roe of sturgeon or salmon fish or calf thymus or chicken blood or pig spleen are ground and homogenized in citrate-salt solution, the homogenate is incubated in the presence of detergent, then under continuous stirring at high temperature concentrated sodium chloride solution is added, reaction mixture is cooled, sonicated, treated with sorbent and detergent, filtrated and concentrated; the amorphous white DNA-Na residue is precipitated from the concentrate with ethanol, the molecular mass of the residue is 270-500 kDa, hyperchromic effect is not lower than 37%, protein contents is lower than 1%. Then the DNA-Na residue is dried and dissolved in water solution of transition metal to obtain 1.5% solution with hyperchromic effect not lower than 10%. Then the preparation is sterilized and poured under sterile conditions in therapeutic doses, as specified in claim 1.

Besides, water-soluble salts of transition metals Zn, Co, Fe, Mn, Sb are used to obtain immunotropic antiviral preparation.

Also DNA-Na to metal ratio in the preparation lays within 50-500:1-2.

At that immunotropic preparation possesses antiviral activity towards RNA- and DNA-viruses.

### Embodiment of the invention

Defatted soft roe of sturgeon fish (1 kg) or defatted soft roe of salmon fish (1 kg) or calf thymus (1 kg) or chicken blood (1 L) or pig spleen (1 kg) are ground in mincer, homogenized in 2 L of citrate-salt solution (0.15M NaCl, 0.015M Na-citrate). The homogenate is placed to the reactor with warmed catrate-salt solution (3 L), 10 L of 6% solution of sodium dodecylsulphate in 45% ethanol are added. The mixture is incubated at 60°C for 1.5 h under continuous stirring. Then the equal volume of 5M NaCl solution is added to the mixture and the process goes on at the same temperature for another 1.5 h. Then the mixture is cooled to room temperature and sonicated for 80 min at 8kHz. The sonicated mass is mixed with kieselguhr at the ratio of 5:1 (volume:mass) and the liquid phase is separated at nutch filter. After micro-filtration the liquid phase is concentrated by baro-membrane method (membrane with pore size of 0.45 µm). DNA-Na is precipitated from the concentrate with 96% ethanol. The precipitate is thoroughly washed with 70% ethanol and dried at 60°C.

The molecular mass of obtained DNA-Na is 270-500 kDa, hyperchromic effect is not lower than 37%, protein contents - lower than 1%. In order to get immunotropic preparation the calculated amount of DNA-Na is weighed and dissolved in water solution of one of the transition metals selected from Zn, Mn, Fe, Co, Sb. The dissolving is carried out under normal temperature, the solution is incubated during 6 hrs. Then sterilization through membranes with pore size of 0.22 µm and sterile pouring in therapeutic doses is carried out.

As it is shown in the table 1 the preparations obtained according to the said method do not exhibit cytotoxicity. Immunotropic properties of the preparation depend on hyperchromic effect that is not lower than 10% in all the examples listed below.

### The preferred embodiments of the invention

### Example 1.

I kg of defatted soft roe of sturgeon fish is ground in mincer, homogenized in 2 L of citrate-salt solution (0.15M NaCl, 0.015M Na-citrate) and placed to the reactor with warmed citrate-salt solution (3 L), 10 L of 6% solution of sodium dodecylsulphate in 45% ethanol are added. The mixture is maintained at 60°C for 1.5 h under continuous stirring. Then the equal volume of 5M NaCl solution is added to the mixture and the process goes on at the same temperature for another 1.5 h. Then the mixture is cooled to room temperature and sonicated for 80 min at 8kHz and intensity, 2 W/cm². The sonicated mass is mixed with kieselguhr at the ratio of 5:1 (volume:mass) and the liquid phase is separated at nutch filter. After micro-filtration the liquid phase is concentrated by baro-membrane method (membrane with pore size of 0.45 µm). DNA-Na is precipitated from the concentrate with 96% ethanol. The precipitate was thoroughly washed with 70% ethanol and dried at 60°C.

### Example 2.

1 kg of defatted soft roe of salmon fish is ground in mincer, homogenized in 2 L of citrate-salt solution and then treated as described in Example 1.

### Example 3.

1 kg of calf thymus is ground and homogenized in 2 L of citrate-salt solution. The process is realized as described in Example 1 up to the stage of barofiltration. Then the liquid phase is concentrated trough the membrane with pore size of 0.8 µm and the process is carried out as described in Example 1.

### Example 4.

1 L of chicken blood is homogenized in 2 L of citrate-salt solution. The process is realized as described in Example 1 but the time of sonication is 10 min at 2 W/cm² and 35 kHz, the ratio of kieselguhr and reaction mixture is 1:25. Then the process is carried out as described in Example 1.

### Example 5.

1 kg of pig spleen is ground and homogenized in 2 L of citrate-salt solution. The process is realized as described in Example 1 up to the stage of barofiltration. Then the liquid phase is concentrated trough the membrane with pore size of 0.8 µm. DNA-Na is precipitated with ethanol, alcohol to concentrate ratio is 1:2. The precipitate is dried at 50°C-60°C.

### Example 6.

The process is realized as described in Example 1 up to the stage of sonication. Then the reaction mass is mixed with kieselguhr (5:1) and filtrated through mrtch filter. DNA-Na is precipitated from the filtrate by twofold re-precipitation with ethanol without preliminary concentration, the powder precipitate is dried at 40°C.

The controlled size of fragments and undamaged state of DNA-Na macromolecule secondary structure and high purification efficiency guarantee high biological activity and pharmacological stability of the preparation.

### Industrial applicability

The stable human cell line MT-4 sensitive to RNA- and DNA-viruses is used to determine antiviral activity of obtained immunotropic DNA-Na preparations containing metal according to table 1.

The cells were grown in RPM 1 1640 medium in concentration of (0.5-0.7)x10⁶ cells in 1 ml of 20% fetal serum. Before the preparation was introduced into cell suspension the cells were infected with concentrate of cultural liquid of H9/IIIB cells producing HIV-1. The infection multiplicity was 0.01 CTD₅₀ per cell. The cultures were incubated at 37°C under 5% CO₂ and 98% humidity atmosphere for 4-7 days, then nutrient medium was changed. The cytopathic effect of the virus in cell culture was determined on the 4-7^{th} day of the experiment. The viability increase up to 70.7-85.2% was observed for the cells preliminary infected with the virus and treated with the preparations in concentrations of 600-1500 mg/ml correspondingly (control of non-infected cells - 89.6%, control of virus-infected cells - 29.3% on the 4^{th} day after infection).

The results of antiviral effect towards HIV-1 estimation for the obtained immunotropic preparations are shown in table 2.

The activity of the preparations presented in table 2 towards human cytomegaloviral infection was studied in vitro on the model of human diploid cells M-19 infected with cytomegalovirus. When the preparations were used in different concentrations the virus titres decreased by 1-2 lg that confirms the antiviral activity of the preparations of the invention.

The antiviral activity of the preparations towards human simplex herpes virus was studied on the model of mice herpes encephalitis caused by this virus. The study of protective effect against lethal herpetic infection was carried out in comparison with protective effect of interferon inducer redostin that is used now as an antiherpetic preparation. The preparations of the invention were introduced once intraperitoneally in the dose of 100 µg/kg 24 hrs before infection. The animals were infected intracerebrally in the dose of 3 lg of LD₅₀. Each group consisted of 30 mice. The results were estimated by survival (in %) of animals to the 15^{th} day and by protective effect (in %) with respect to survived animals in control group. To the 15^{th} day 96% of control mice died whereas 70% of mice survived in groups that were given preparation obtained according to the invention.

Considering that protective effect against lethal herpetic infection is observed under severe conditions of the experiment - intracerebral infection, high infective dose of the virus, single peripheral dosing of the preparation- it is possible to say that the preparation obtained according to the invention possesses moderately expressed antiherpetic effect.

Immunotropic properties of the preparations of the invention were determined by morphometric method. The essence of the method is following. The immunodeficiency state was generated in mice by single intraperitoneal introduction of hydrocortisone acetate in the dose of 0.2 mg/mouse. 48 hrs after introduction single intraperitoneal injections of the DNA-Na/Me preparations were made in super small doses during 3 days. The experimental study of the immunological activity of the preparations was carried out on 154 white mice and 97 hybrid CBA mice of both sex of 18-24 g in weight. The dosage was determined according to literary data (Havenson V.Kh., Zhukov V.V. "Thymus peptides and immunomodulation mechanisms", Modem biology achievements, v. 112, iss. 4, pp. 554-570). Physiological solution was used as a control. The animals were slaughtered after 18, 36, 60 and 84 hours. Immimotropic properties appear in relative thymus mass decrease and as a rule in relative spleen mass increase.

The experiment results show that relative thymus mass decreases by 26% not depending on the preparation dose. No reliable difference in spleen mass was detected.

The effect of small doses of the preparation on thymus may be considered as the most characteristic factor of increase in mice immune system functional activity. The results of the experiment are shown in table 4.

As it follows from the table DNA-Na-Fe preparation possesses evident immunotropic activity appearing in its ability to decrease mice relative thymus mass in small doses (1*10⁻⁵ g/ml, 1*10⁻⁷g/ml, 1*10⁻⁹ g/ml), dose dependence being not detected. The preparation effect is marked after 18 hours (20.7%), maximum peak falls on 36 hours (28.6%), and full normalization comes after 80 hours. DNA-Na-Sb, DNA-Na-Co, DNA-Na-Zn, DNA-Na-Mn possess the same immunotropic properties.

According to proposed method of obtaining immunotropic antiviral preparations it is necessary to fulfill the process in stated order. It guarantees long-term stability of immunomodulating and antiviral properties of the preparations obtained according to proposed method.

**Table 1**

| Metal | Metal/DNA-Na ratio | Cytotoxicity results In Vitro | |
|---|---|---|---|
| | | Cells in 1 ml of culture x10⁶ | Cells viability, % |
| Zinc | 1/50 | 0.89 | 0.86 |
| | 2/50 | 0.89 | 0.80 |
| | 1/150 | 0.89 | 0.83 |
| | 1/500 | 0.89 | 0.87 |
| Iron | 1/50 | 0.89 | 0.88 |
| | 2/50 | 0.89 | 0.86 |
| | 1/150 | 0.89 | 0.82 |
| | 1/500 | 0.89 | 0.87 |
| Cobalt | 1/50 | 0.89 | 0.81 |
| | 2/50 | 0.89 | 0.79 |
| | 1/150 | 0.89 | 0.81 |
| | 1/500 | 0.89 | 0.82 |
| Manganese | 1/50 | 0.89 | 0.86 |
| | 2/50 | 0.89 | 0.80 |
| | 1/150 | 0.89 | 0.82 |
| | 1/500 | 0.89 | 0.82 |
| Stibium | 1/50 | 0.89 | 0.84 |
| | 2/50 | 0.89 | 0.80 |
| | 1/150 | 0.89 | 0.82 |
| | 1/500 | 0.89 | 0.85 |

**Table 2**

| DNA-Na-Me preparation 1:200 800 µg/ml | Cells viability, % | | | | Syncytium formation Cytopathic effect of the virus | | Immune-enzyme analysis, optical density |
|---|---|---|---|---|---|---|---|
| | Non-infected | | Infected | | | | |
| | Day | | | | | | |
| | 4th | 7th | 4th | 7th | 4th | 7th | |
| Cells control | 89.6 | 82.3 | - | - | - | - | 0.104 |
| Virus control | - | - | 29.3 | 2.4 | ++++ | ++++ | 0.737 |
| DNA-Na-Fe | 87.5 | 84.6 | 78.6 | 35.4 | + | ++ | 0.248 |
| DNA-Na-Zn | 83.1 | 81.9 | 60.0 | 30.1 | ++ | ++ | 0.326 |
| DNA-Na-Co | 86.0 | 80.5 | 79.1 | 76.7 | ++ | ++ | 0.285 |
| DNA-Na-Sb | 83.5 | 75.3 | 68.0 | 29.7 | ++ | ++ | 0.487 |
| DNA-Na-Mn | 84.1 | 80.4 | 70.5 | 31.3 | + | ++ | 0.236 |
| Azidothymidine 1.0 | 74.2 | 49.1 | 57.1 | 23.8 | - | ++ | 0.186 |

**Table 3**

| Mice herpes encephalitis, type 1, L2 strain | | | | |
|---|---|---|---|---|
| Preparation | Dose mg/kg | Scheme of intraperitoneal introduction | Survival % | Protective effect % |
| 1 | 2 | 3 | 4 | 5 |
| DNA-Na-Fe | 100 | 24 hrs before infection | 23.5 | 20 |
| DNA-Na-Sb | 100 | The same | 20 | 18 |
| DNA-Na-Co | 100 | The same | 25 | 22.3 |
| DNA-Na-Zn | 100 | The same | 18 | 15 |
| DNA-Na-Mn | 100 | The same | 15 | 12 |
| Redostin | 5 | The same | 30 | 28 |

**Table 4**

| Time | Thymus (% of body weight) | | Spleen (% of body weight) | |
|---|---|---|---|---|
| | Experiment DNA-Na-Fe | Control (physiological solution) | Experiment DNA-Na-Fe | Control (physiological solution) |
| 18 hrs | 0.230±0.009* | 0.29±0.021 | 0.501±0.027 | 0.490±0.026 |
| | n=18 | n=18 | n=18 | n=18 |
| 36 hrs | 0.253±0.03** | 0.353±0.014 | 0.591±0.045 | 0.506±0.036 |
| | n=20 | n=20 | n=20 | n=20 |
| 60 hrs | 0.28±0.018* | 0.35±0.023 | 0.52±0.05 | 0.54±0.038 |
| | n=20 | n=20 | n=20 | n=20 |
| 84 hrs | 0.30±0.019 | 0.304±0.015 | 0.61±0.053 | 0.53±0.038 |
| | n=19 | n=19 | n=19 | n=19 |

| | | | | |
|---|---|---|---|---|
| *- p<0.05 for experiment in comparison with control | | | | |
| **- p<0.01 for experiment in comparison with control | | | | |

## Claims

1. A method of obtaining immunotropic antiviral apyrogenic non-toxic preparations based on low-molecular sodium salt of native deoxyribonucleic acid (DNA-Na) comprising grind and homogenization of animal material in citrate-salt solution, homogenate incubation in the presence of detergent, sodium chloride solution adding, sonication, subsequent purification and isolating of the desired product, wherein defatted soft roe of sturgeon fish or salmon fish, or calf thymus, or chicken blood, or pig spleen are used as an animal material, reaction mix is sonicated for 80 minutes at 8 kHz-35 kHz, filtrated and concentrated by baromembrane method, DNA-Na white amorphous precipitate is isolated with 96% ethanol, DNA-Na molecular mass is 270-500 kD, hyperchromic effect is not lower than 37%, protein content is less than 1 %, then DNA-Na precipitate is dried and dissolved in standardized solution of water-soluble salt of transition metal selected from Zn, Co, Fe, Mn or Sb to make 1.5% pharmaceutical immunotropic complex DNA-Na-Me with hyperchromic effect not lower than 10%, then it is sterilized and poured.

2. A method of claim 1, wherein DNA-Na to metal ratio in the preparation is within range 50-500 : 1-2.

3. A method of claim 1. wherein immunotropic preparation possesses antiviral activity towards RNA- and DNA-viruses and their combined presence.

4. An immunotropic antiviral apyrogenic preparation obtainable by a method of any of claims 1 to 3, **characterized in that** the preparation is 1.5% complex DNA-Na-Me in the solution of water-soluble salt of transition metal selected from Zn, Co, Fe, Mn and Sb, and the said complex has hyperchromic effect not lower than 10% and ratio DNA-Na to metal is within 50-500 : 1-2, wherein the said preparation possesses antiviral activity towards RNA- and DNA-viruses including their combined presence.

5. Use of a preparation of claim 4 for the preparation of a medicament or a vaccine against RNA- and DNA-viruses.

## Patentansprüche

1. Verfahren zur Herstellung von immunotropen apyrogenen nichttoxischen Antiviruspräparaten auf der Basis von niedermolekularem Natriumsalz einer nativen Desoxyribonukleinsäure (DNS-Na), umfassend Zermahlen und Homogenisieren eines tierischen Materials in einer Zitratsalzlösung, Inkubation des Homogenisats in Gegenwart eines Detergens, Zugabe einer Natriumchloridlösung, Ultraschallbehandlung mit nachfolgender Reinigung und Isolierung des Endproduktes, **dadurch gekennzeichnet, dass** es sich bei dem tierischen Material um entfettete Milch von Stör- oder Lachsfischen oder Kalbthymus oder Hühnchenblut oder Schweinmilz handelt, das Reaktionsgemisch 80 Minuten lang mit Ultraschall bei 8 bis 35 kHz behandelt, gefiltert und nach der Baromembranmethode konzentriert wird, ein weißer amorpher DNS-Na-Niederschlag mit einer DNS-Na-Molekularmasse 270 bis 500 kD, einem hyperchromen Effekt von mindestens 37% und einem Eiweissgehalt weniger 1% mit 96%-igem Äthanol isoliert wird, der DNS-Na-Niederschlag daraufhin getrocknet und in der standardisierten Lösung eines wasserlöslichen Salzes eines Übergangsmetalls aus der Reihe Zn, Co, Fe, Mn oder Sb aufgelöst wird unter Herstellung eines 1,5%-igen pharmazeutischen immunotropen DNS-Na-Me-Komplexes mit dem hyperchromen Effekt von mindestens 10% und abschließend sterilisiert und abgefüllt wird.

2. Verfahren nach Anspruch 1, **dadurch gekennzeichnet, dass** das DNS-Na-zu-Metall-Verhältnis im Bereich 50-500 : 1-2 liegt.

3. Verfahren nach Anspruch 1, **dadurch gekennzeichnet, dass** das immunotrope Präparat gegen RNS- und DNS-Viren und deren gemeinsame Anwesenheit wirksam ist.

4. Immunotropes apyrogenes Antiviruspräparat, herstellbar nach einem Verfahren gemäß einem der Ansprüche 1 bis 3, **dadurch gekennzeichnet, dass** das Präparat ein 1,5%-iger DNS-Na-Me-Komplex, gelöst in einem wasserlöslichen Salz eines Übergangsmetalls aus der Reihe Zn, Co, Fe, Mn oder Sb ist, der genannte Komplex einen hyperchromen Effekt von mindestens 10% und das DNS-Na-zu-Metall-Verhältnis im Bereich 50-500 : 1-2 aufweist, wobei das genannte Präparat gegen RNS- und DNS-Viren, darunter bei deren gemeinsamer Anwesenheit wirksam ist.

5. Verwendung des Präparats nach Anspruch 4 für das Zubereiten eines Arzneimittels oder einer Vakzine gegen RNS- und DNS-Viren.

## Revendications

1. Procédé d'obtention des préparations immunotropes antivirales apyrogènes non-toxiques à la base de sel de sodium bas-moléculaire de l'acide native désoxyribonucléique (DNA-Na), comprenant le bocardage et l'homogénéisation du matériau animal dans la solution saline de citrate, l'incubation d'homogénate en présence d'un détergent, l'addition de solution de chlorure de sodium, l'ultrasonnation, la purification subséquente et l'isolation du produit désiré, **caractérisé en ce qu'**on utilise comme matériau animal des laitances dégraissées des poissons d'esturgeon et de saumon, ou bien le thymus du veau, ou bien le sang des poulets, ou bien le rate du cochon, le mélange réactionnel étant ultrasonné pendant 80 minutes à la fréquence égale à 8 KHz - 35 KHz, étant filtré et concentré par la méthode baromembrane, on isole du concentré par éthanol 96% le précipité amorphe blanc de DNA -NA à la masse moléculaire 270 - 500 KD, à l'effet hyperchrome pas moins de 37% et à la teneur en protéine moins de 1%, puis on sèche le précipité de DNA-Na et on dissout la poudre dans la solution standardisée de sel soluble dans l'eau du métal de transition choisi parmi Zn, Co, Fe, Mn et Sb avec obtention du 1,5% complexe pharmaceutique ummunotrope de DNA-Na-Me, dont l'effet hyperchrome est pas moins de 10%, on stérilise et on verse.

2. Procédé selon la revendication 1, **caractérisé en ce que** dans la préparation le rapport DNA - Na : métal est égal à 50-500 : 1-2.

3. Procédé selon la revendication 1, **caractérisé en ce que** la préparation ummunotrope manifeste l'activité antivirale envers virus contenant RNA et DNA et à leur présence combinée.

4. Préparation immunotrope antivirale apyrogène obtenue par la méthode selon l'une des revendications 1 - 3, **caractérisée en ce que** la préparation représente le 1,5% complexe de DNA-Na-Me dans la solution de sel soluble dans l'eau du métal de transition choisi parmi Zn, Co, Fe, Mn et Sb et ledit complexe a un effet hyperchrome pas moins de 10% et le rapport DNA - Na : métal est égal à 50-500 : 1-2, ladite préparation manifestant une activité antivirale envers virus contenant RNA et DNA et à leur présence combinée.

5. Utilisation de la préparation selon la revendication 4 pour préparation de médicament ou de vaccin contre virus contenant RNA et DNA.
